# EUROPEAN PATENT APPLICATION

(11) **EP 1 880 738 A1**
(43) Date of publication of application: **23.01.2008**
(21) Application number: 06732543.1
(22) Date of filing: 12.05.2006
(51) Int. Cl.: A61L 24/00, A61K 9/06, A61K 31/198, A61K 31/405, A61K 31/4172, A61K 31/7004, A61K 47/36, A61P 17/02

(54) **MEDICAL COMPOSITION FOR PROMOTION OF SKIN REGENERATION**

(30) Priority: 13.05.2005 JP 2005140982
(71) Applicant: Netech Inc., Kanagawa 213-0012 (JP); Yaizu Suisankagaku Industry Co., Ltd., Yaizu-shi, Shizuoka 425-8570 (JP)
(72) Inventor: KANATANI, Yasuhiro, 3591111 (JP); KIYOZUMI, Tetsuro, Tokorozawa-shi Saitama 3590042 (JP); OKADA, Yoshiaki, 1650031 (JP); SAITOH, Daizoh, Tokorozawa-shi Saitama 3590042 (JP); ISHIHARA, Masayuki, 1900033 (JP); YURA, Hirofumi, Kawasaki-shi Kanagawa 2130011 (JP); MISAWA, Yoshinori, 4250036 (JP)
(74) Representative: O'Neill, Michelle
(86) International application number: PCT/JP2006/309562
(87) International publication number: WO 2006/121156

(57) **Abstract**

The present invention provides a medical composition which, in autotransplantation which is the only method that can induce the epithelialization even in a widespread full-thickness skin defect, enables the fixation of an autotransplantation skin graft in a simple manner and can increase the efficiency of epithelialization to promote the regeneration of the skin. The present invention elates to a medical composition comprising a photo-crosslinkable chitosan derivative and an amino acid and/or a saccharide. The amino acid is preferably an essential amino acid, and the saccharide is preferably a neutral saccharide selected from glucose, galactose, mannose and fucose.

## Description

### TECHNICAL FIELD

The present invention relates to a medical composition capable of promoting skin regeneration, comprising a photo-crosslinkable chitosan derivative and a saccharide, such as glucose and/or amino acids, such as glycine.

### BACKGROUND ART

Skin intrinsically has the capacity to repair for itself unaided. Therefore, in the case of a mild wound, such as a simple external injury, the skin can be regenerated by its self-repair function. However, in the case of refractory wounds, such as serious burns, complex wounds from radiation exposure, and decubitus, it is difficult to completely regenerate the skin (non-patent document 1).
The process of wound healing includes the following steps: (1) recognition of the damaged area by the inflammatory cells, and subsequently by the connective tissue cells and epidermal cells; (2) shrinkage of the wound area; and (3) granulation and epithelialization. The cells, various factors, cytokines and secretions involved in each stage of this process have been identified.

The study of artificial skin reparation began with just modifying materials which cover the wound for temporary protection of the wound surface, and now, has reached to induction of more aggressive treatments by using agents such as cytokines and growth factors that are related to wound healing. However, there has never been a coating or a single component agent which is effective for various degrees of skin wounds, from simple to refractory. In particular, there is no technique capable of facilitating and inducing epithelialization that is especially important for the treatment of serious bums and large area wounds.

Under these circumstances, it has begun to use a skin substitute for the treatment in patients who have complete loss of full-thickness skin. The skin substitute incorporating epidermal cells is called as cultured epidermis, the skin substitute incorporating dermis fibroblasts is called as cultured dermis, and the skin substitute incorporating both of them is called as cultured skin.
For example, frozen cultured epidermis is used for the treatment of bums having a depth degree of II, and the homologous cultured epidermis changes to autologous cells. However, although facilitation of granulation may lead to epithelialization even in full-thickness skin cruris ulcers or burn having a depth degree of III if the area of complete loss of full-thickness skin is small. However, if the area of complete loss of full-thickness skin is large, adhesion of cultured epidermis is low, and ultimately, there is no other choice but to rely on autodermic graft.

Currently, as cultured dermis, some products having different matrixes into which fibroblasts are incorporated, such as TransCyte (trade name) and Dermagraft (trade name), are available from Smith & Nephew PLC. However, cultured dermis, as well as cultured epidermis, do not have an ability to induce epithelialization in large wounds, and therefore, cultured dermis do no better than functional wound dressing.
On the other hand, as cultured skin incorporating epidermal cells and fibroblasts, Apligrag (trade name) (NOVARTIS Pharma K.K.) and VivoDerm (trade name) are available (ConvaTec, A Bristol-Myers Squibb Company). However, from a practical point of view, there are some problems regarding the affinity between cultured epidermal layer and dermal layer, and the insufficiency in clinical effect obtainable against an infected wound. Therefore, it is far remove from the establishment of a skin substitute feasible to completely replacement with autologous skin.

In addition, there is a concern for the safety of the current skin substitutes that are highly dependent on animal collagen or human plasma components.
Furthermore, although artificial dermis have been used for the treatment of serious burns, repeating autodermic graft is necessary for epithelialization finally. In addition, grafting only cultured skin to serious bums is not effective, and although there are some facilities that study hybrid cultured skin combined with non-cellular dermis, but clinical evaluation of the hybrid cultured skin has not been established.

Most functional skin regeneration and treatment technique is predominantly needed in cases of complete loss of full-thickness skin in which no appendages of skin is remained, as in large area serious burns, and the key to successful treatment is rapid formation of the dermis and epithelialization. However, there is currently no skin substitute that has the ability to achieve epithelialization. Accordingly, the only way to induce epihelialization is currently autodermic grafting. However, if the burn area is large, the skin to be collected for autodermic grafts is limited, and therefore, it is difficult to secure sufficient quantity of skin necessary for epithelialization to cover the whole burn area. In addition, use of sutures and staples to adhere grafted tissue to the hypodermal tissue takes time, and involves the risk of pain during bandage changes after grafting and reopening the wound, which foist a large burden to the patients and doctors.
Non-patent document 1: Kenji Takayanagi, Norio Kumagai "Protein, Nucleic acid and Enzyme" Vol. 45, No.13, Pages 2283- 2287 (2000).

### SUMMARY OF THE INVENTION

### PROBLEM TO BE SOLVED BY THE INVENTION

The problem in the present invention is to provide a medical composition that enables autodermic grafts to be easily adhered so that skin regeneration can be facilitated by enhancing epithelialization in autodermic grafting, which is the only epithelialization method usable for large area loss of full-thickness skin, such as serious bums. Particularly, in the treatment of serious burns, it has been recognized that facilitating granulation in an early stage largely affect adhesion of autodermic grafts, and therefore, many burn specialists have been awaiting the development of a simple high polymer skin agent which can facilitate granulation.

### MEANS FOR SOLVING THE PROBLEM

To resolve the problem, the present invention provides a medical composition characterized by containing a base material and a saccharide and/or amino acid. As the base material, a dressing material which supports repairing wounds, healing and regeneration of tissue is preferably employed. Examples include a variety of hydro-gel, hydrocolloid, collagen, and gelatin preparations. It is especially preferable to use hydro-gel made from photo-crosslinkable chitosan derivative, which will be discussed below.

### EFFECT OF THE INVENTION

Photo-crosslinkable chitosan derivative (PRC) used in this invention may be selected from, for example, those described in the WO00/27889 pamphlet, and is a functional polymer becoming adhesive hydro-gel with approximately 400 nm safety UV and also suitable as a medical adhesive. Therefore, the composition of the present invention comprising the PRC has not only similar basic features that PRC has, which are preventing infection of the wound area by adhering and sealing graft tissues such as autodermic graft with easy operation and preserving the active granulation ability inherently to the living tissue, but also characteristics that is especially suitable for the intended use of this invention including burn treatment, which is eliminating problems of impediment during replacement due to early decomposition.

Even more surprising, by coexisting amino acid and/or saccharide in the medium in which PRC is dissolved, polynuclear globus, predominantly neutrophils, smoothly infiltrates into the chitosan layer and VEGF appearance is promoted by the polynuclear globus. With this appearance, facilitation of new blood vessel formation, granulation and epithelialization was observed in the damaged areas of tissues as well as in the chitosan gel as a support material (with dissolving of the PRC).
By admixing the wound healing promoter, such as a cell growth factor, into PRC, the wound healing activity can be increased (refer to WO03/090765 pamphlet). However, the composition of the present invention enables adhesion of autodermic grafts and other skin substitutes to the wound area without containing growth factors, and can be used not only as a support material for skin substitutes in skin wounds such as serious burns, but also as a skin wound treatment agent with the effect of promoting healing by facilitating epithelialization even without skin grafts or skin substitutes.
In addition, since the composition of the present invention is not derived from human tissue, such as conventional adhesives including fibrin adhesives and collagen preparations, the composition also has the advantage of not being at risk of infection.

### BRIEF DESCRIPTION OF THE DRAWINGS

[Fig.1] Explanation figure showing the outline of autodermic graft experiments in the Example.
[Fig.2] Microscopic view showing the tissue change in the wound area when composition (A) of this invention is used in Example 2.
[Fig.3] Microscopic view showing the tissue change in wound area when composition (B), that does not contain amino acid and saccharide, is used in Example 2.
[Fig.4] Microscopic view showing the cross-section of anti-VEGF stained tissue in wound area when composition (A) of this invention is used in Example 3
[Fig.5] Microscopic view showing the cross-section of anti-VEGF stained tissue in wound area when composition (A) of this invention is used in Example 3
[Fig.6] A Photograph showing artificial burn formation and treatment methods in Example 6.
[Fig.7] A Graph showing the change of thickness in the granulation tissue in a burn area in Example 6.
[Fig.8] A Graph showing the change of the number of blood capillaries in a burn area in Example 6.

### BEST MODE FOR CARRYUNG OUT THE INVENTION

The photo-crosslinkable chitosan derivative used in the medical composition of the present invention has a structure in which a carbohydrate chain and a photo-crosslinkable group are introduced into a polymer backbone, which is generally called as chitin/chitosan. In particular, those formed by incorporating a carbohydrate having reducing terminals and a photo-reactive functional group to at least a part of the 2-position amino groups in the glucosamin units constituting an at least partially deacetylated chitin/chitosan are preferable.

Normally, chitin/chitosans are deacetylated acid-soluble fractions obtained by alkali processing chitin (poly-N-acetylglucosamins) originated from crab shells, and generally have the constituent units expressed by the following formulas (1) and (2). Otherwise, there is no problem in using chitin/chitosans derived from cartilage of cuttlefish, insects or plants.

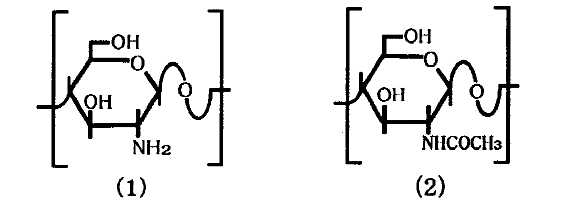

Among chitin/chitosans, some persons call those having a low degree of deacetylation (normally less than 40%) as "chitins" and those having a high degree of deacetylation (normally 40% or more) as "chitosans", but henceforth in the present specification, all chitin/chitosans which are at least partially deacetylated shall be referred to collectively as "chitosans". Additionally, in the present invention, chitosans are not limited to those of natural origin, and may be chemically modified carbohydrate chains having similar structures synthesized chemically or by genetic engineering.
Here, "degree of deacetylation" refers to the proportion of acetylamino groups in the 2-position of the carbohydrate units constituting the chitosan (or poly-N-acetylglucosamin), which have been converted to free amino groups by deacetylation. In the present specification, the degree of deacetylation is measured by means of the "colloidal titration method" described in "Health Foods Standard and Criterion (No. 4)", Japan Health Food and Nutrition Food Association (1996), p. 55.

The chitosan derivative of the present invention has been functionalized by further chemically modifying the chitosan, and the chitosan used as the raw material should preferably have a degree of deacetylation of at least 40%, preferably 60-100%, more preferably 65-95%. A chitosan having a 100% degree of acetylation consists entirely of the constituent units of the above-given formula (1), and does not include the constituent units of formula (2).
Additionally, there are no particular restrictions on the molecular weight of the chitosan, and this can be changed of a wide range depending on the projected use of the chitosan derivative, but in general, the number-average molecular weight should be in the range of 5,000-2,000,000, preferably 10,000-1,800,000, more preferably 40,000-1,500,000.

The chitosan derivatives suitable for the present invention are those formed by incorporating a carbohydrate having reducing terminals to at least a portion of the 2-position amino groups in the glucosamin units (1) constituting the above-described chitosan and a photo-reactive functional group to at least another portion of the 2-position amino groups. Details of such chitosan derivatives are described in WO00/27889 pamphlet.

The carbohydrates having reducing terminals to be incorporated to the chitosan derivatives include aldoses and ketoses, among which those having 20 or less constituent carbohydrate units, especially those with 1-7 units are preferably used. Specific examples include pentaoses and hexaoses such as glucose, fructose, galactose, fucose, mannose, arabinose, xylose, erythrose, hepturose and hexylose, amino carbohydrates such as glucosamin, N-acetylglucosamin and galacsamin; carbohydrate derivatives such as uronic acids and deoxysaccharides; di- and trisaccharides such as maltose, isomaltose, lactose, melibiose and maltotriose composed of carbohydrate chains combining the above-mentioned monosaccharides; and the various oligosaccharides, among which the neutral disaccharides such as maltose, lactose and melibiose are preferable. Glucose is particularly preferable.
While it is also possible to derive chitosans from organic compounds such as polyethers and polyhydric alcohols instead of the above-mentioned carbohydrates, it is preferable to use natural carbohydrate chains in consideration of biocompatibility.

The incorporation of the above-mentioned carbohydrates in the 2-position amino group of the glucosamin units of the chitosan of the above-given formula (1) can itself be performed using known methods. For example, methods of carboxylating the reducing terminal of a carbohydrate, then binding to the 2-position amino group by an amide bond (see, for example, Japanese Patent Application, First Publication No. H10-120705), or of aldehydating or carbonylating the reducing terminal of a carbohydrate, then binding to the 2-position amino group of a glucosamin unit by a reduction alkylation method by means of a Schiff base (see, for example, "Applications of Chitins and Chitosans", edited by Chitin/Chitosan Workshop, pp. 53-56, Feb. 20, 1990, published by Gihodo Shuppan KK).
The carbohydrate incorporated in the chitosan in the present invention is not limited to only one type, and it is possible to use a combination of 2 or more.

Specific examples of a carbohydrate side chain constituting the chitosan derivative of the present invention include the following, but there is no restriction to these.

(i) Carbohydrate derived from lactose: 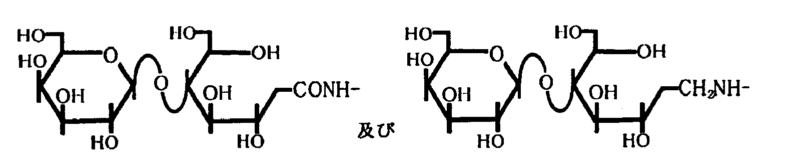
(ii) Carbohydrate derived from maltose: 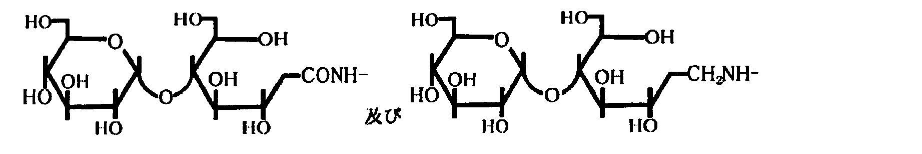
(iii) Carbohydrate derived from melibiose: 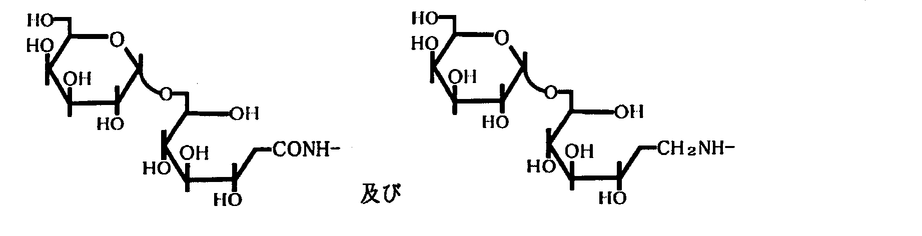
(iv) Carbohydrate derived from cellobiose: 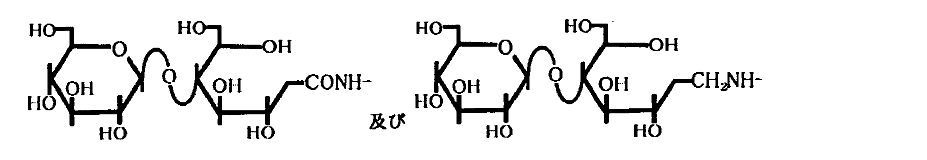
(v) Carbohydrate derived from laminalibiose: 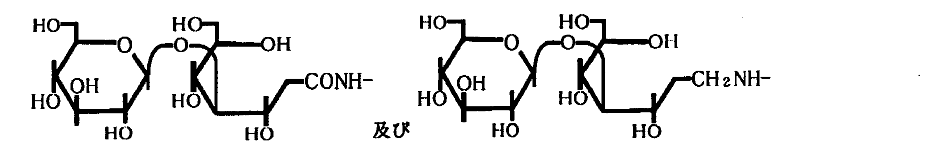
(vi) Carbohydrate derived from mannobiose: 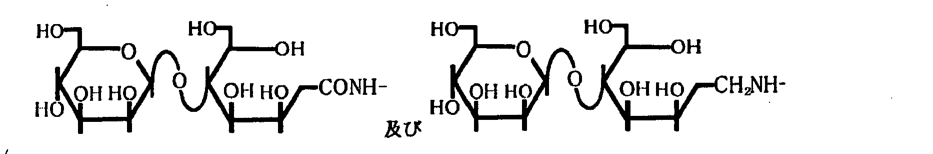
(vii) Carbohydrate derived from N-acetylchitobiose: 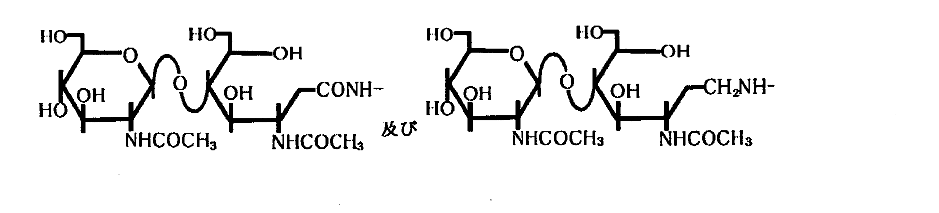
Of the carbohydrate side chains given in the above (i)-(vii), those on the left side represent residual groups incorporated by means of condensation between a carboxyl group on the carbohydrate and a 2-position amino group on the chitosan, while those on the right side represent residual groups bound by a Schiff base.
The acid-depending solubility of the chitosan is relieved by introducing the carbohydrate chains to the 2-position of the glucosamine unit of chitosan, and solubilization at neutral region can be accomplished.
While the degree of substitution of 2-position amino groups in the glucosamin units of chitosan by carbohydrate side chains can be changed depending on the physical properties desired in the final chitosan derivative, the degree of substitution should generally be in the range of 0.1-80%, preferably 0.5-60%, more preferably 1-40%. Here, the "degree of substitution" of the carbohydrate side chain is the level to which the amino groups in the 2-position of the carbohydrate units constituting the chitosans are substituted by carbohydrate side chains, and denote the proportion of substituted amino groups with respect to the total number of free amino groups and substituted amino groups at the 2-position of the carbohydrate units constituting the chitosans. In the present specification, the degree of substitution of carbohydrate side chains is measured by the "phenol -sulfuric acid method" wherein the characteristic color emission due to a reaction between carbohydrate chains and phenol in sulfuric acid is sensed by light absorption at 490 nm (see J. E. Hodge, B. T. Hofreiter, "Methods in Carbohydrate Chemistry", ed. by R. L. Whistler, M. L. Wolfrom, vol. 1, p. 388, Academic Press, New York (1962)).

In addition, the chitosan derivative of the present invention has a self-crosslinking property by photo-irradiation due to incorporating photo-reactive functional groups in the 2-position amino groups in the glucosamin units of the above-given formula (1) constituting the chitosan.
The photo-reactive functional groups used for chemical modification of the chitosans according to the present invention are groups which react with each other and/or amino groups or hydroxyl groups present in the chitosan upon irradiation by ultraviolet light including the near-ultraviolet region of 200-380 nm to form crosslinking bonds including, for example, those derivable from cyclic unsaturated compounds such as benzophenones, cinnamic adds, azides, diolefins and bis-anthracene, especially preferable being those having carbonylazide groups, sulfonylazide groups and aromatic azide groups.
The photo-reactive group may be a substitutional group which reacts by irradiation of visible light of about 400 to 500 nm. Such visible-light-reactive groups include, for example, formyl styryl group represented by the following formula and described in Journal of Polymer Science: Polymer Chemistry Edition, Vol. 20, 1419-1432 (1982). 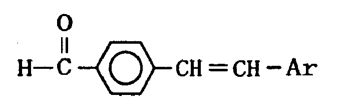 (In this formula, Ar denotes a heterocyclic ring such as pyridin, alkylpyridinium salt, quinolin, or alkylquinolinium salt.)

The incorporation of photo-reactive functional groups to the amino groups at the 2-position in the glucosamin units of the chitosans can itself be performed by known methods, for example, by a method of binding an azide compound having a carboxyl group to the 2-position amino group in the presence of a condensing agent (see Japanese Patent Application, First Publication No. H10-120705); or a method of reacting the azide compound with the 2-position amino group by means of an acid chloride group, an aldehyde group, an N-hydroxysuccinic add imide ester group or an epoxy group (see "Applications of Chitins and Chitosans", edited by Chitin/Chitosan Workshop, pp. 53-5645-65, Feb. 20, 1990, published by Gihodo Shuppan KK). The above-described formyl styryl compound can be incorporated by coupling its formyl group with the amino group of chiotosan.
In azide group crosslinking reactions, it has been conventionally held to be effective to use polyfunctional compounds such as bis-azides or above (see Japanese Patent Application, First Publication No. H9-103481), this is not necessary in the present invention, so that a chitosan derivative having adequate self-crosslinking ability can be obtained by incorporation of monoazide compounds.

Specific examples of a photo-reactive group forming the chitosan derivative of the present invention include, for example, those expressed by the following formulas (A) through (E). The group of formula (A) is derived from p-azidobenzoic acid, the group of formula (B) is derived from p-azidobenzaldehyde, the group of formula (C) is derived from p-benzoylbenzoic add, the group of formula (D) is derived from cinnamic acid, and the group of formula (E) is derived from 1-methyl-4-[2-formylphenyl]ethenyl]pyridinium.

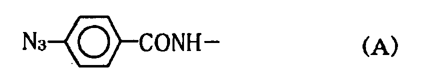

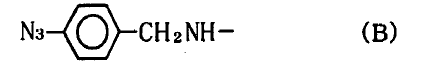

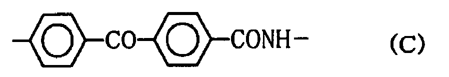

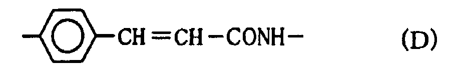

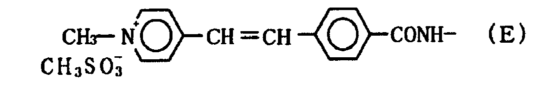

While the degree of substitution of these photo-reactive functional groups can be changed according to the degree of gelification (insolubility) due to the crosslinking reaction desired in the final chitosan derivative, but it is preferable for the degree of substitution of the photo-reactive functional groups to be within the range of 0.1-80%, preferably 0.5-50%, more preferably 1-30%. Here, the "degree of substitution" of the photo-reactive functional groups is the degree of substitution of the 2-position amino groups of the carbohydrate units forming the chitosans with photo-reactive functional groups, and is the proportion of substituted amino groups with respect to the total number of free amino groups and substituted amino groups at the 2-position of the carbohydrate units forming the chitosans. In the present specification, the degree of substitution of photo-reactive functional groups such as azide groups can be determined based on calibration curves obtained from characteristic absorption at 270 nm for 4-azidobenzoic acid.
The degree of substitution of the total of carbohydrate side chains and photo-reacfive functional groups in the chitosan derivatives of the present invention is not particularly restricted, and may vary over a considerable range, but is usually in the range of 0.2-80%, preferably 1.5-65%, more preferably 3-50%.

Additionally, according to the present invention, considerably improved water retention ability of the cross-linked matrix can be obtained by incorporating an amphipathic group to at least a portion of the 3- or 6-position hydroxyl groups in the carbohydrate units of formulas (1) and (2), and the amino groups in the 2-position of the carbohydrate units of formula (1) constituting the chitosan. These amphipathic groups are groups having a hydrophobic block comprising a hydrophobic group and a hydrophilic block comprising a hydrophilic group, and often have a surfactant function. Among these those in which the molecular weight ratio between the hydrophobic blocks (X) and the hydrophilic blocks (Y) is X : Y = 1 : 5 to 5 : 1 are preferably used, and non-ionic groups without dissociated ionic groups are more preferably used. In particular, those composed of a hydrophobic alkyl block and a hydrophilic polyoxyalkylene block and with a molecular weight of at least 90 are preferable, a polyoxyalkylene alkyl ether of 500-10,000 being more preferable. While a polyether not having a hydrophobic block may be used, a polyoxyalkylene alkyl ether is preferable for having both a hydrophobic block and a hydrophilic block in consideration of the improvement to the water retaining ability.
The incorporation of these amphipathic groups to the chitosan can be performed, for example, by a method of incorporating a compound having groups capable of reacting with amino groups to form covalent bonds, such as aldehyde groups or epoxy groups to a terminal portion of either the hydrophilic block or hydrophobic block of the amphipathic group, then reacting with the 2-position amino group of the glucosamin of the chitosan, a method of inducing a reaction between a polyoxyalkylene alkyl ether derivative having a carboxyl group with the chitosan in the presence of a condensing agent, or a method of inducing a reaction between a polyoxyalkylene alkyl ether derivative having an acid chloride group with a hydroxyl group or amino group in the chitosan.

For example, when incorporating a polyoxyalkylene alkyl ether group with an epoxy group on its terminal into an amino group in the chitosan, the amphipathic group is expressed by the following formula (a), and when incorporating a polyoxyalkylene alkyl ether group with an aldehyde group on its terminal into an amino group of the chitosan, the amphipathic group is expressed by the following formula (b). Additionally, when binding a polyoxyalkylene alkyl ether group with an acid chloride group on its terminal to the 3- or 6-position hydroxyl group of the chitosan, the amphipathic groups are expressed by the following formula (c). In the below formulas (a)-(c), n and m are repeating units numbering 1 or more.

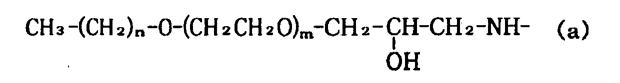

CH₃-(CH₂)ₙ-O-(CH₂CH₂O)ₘ-CH₂-CONH- (b)

CH₃-(CH₂)ₙ-O-(CH₂CH₂O)ₘ-CH₂-CO-CH₂- (c)

The degree of incorporation of amphipathic groups in the chitosan derivatives of the present invention is not particularly restricted, but should be within the range normally of 5-70%, preferably 15-55% based on the change in weight of the chitosan derivative after incorporation.

As described in detail above, in the photo-crosslinkable chitosan derivative used for the medical composition of the present invention, carbohydrates having a reducing terminus and a photoreactive group may be introduced into the chitosan backbone structure, and amphipathic groups can be introduced therein as desired. By introducing the carbohydrate, the chitosan derivative becomes well soluble in neutral regions, can be made into a solution by a physiological buffer or a culture media, and can be mixed without losing the activity of drugs, such as proteins, that may get denatured by acid or alkali. Further, by introducing the photoreactive group, an insoluble gel body may be formed immediately by light irradiation after application to an appropriate region, which adheres a graft such as skin substitute to tissues, and facilitates skin regeneration by promoting epithelialization.

As the polymer constituting the backbone structure of the photo-crosslinkable chitosan derivative of the present invention, instead of chitosan, polysaccharides such as hyaluronic acid, proteins such as collagen, and other synthetic polymers and the like can be used, but carbohydrates capable of sealing wounds and tissues, having drug holding characteristics and appropriate biodegradability, and having an ability to conduct controlled release of a drug at a speed which is not too fast are most suitable. Among these, chitosan, which itself has wound healing characteristics and antibiotic action, or carbohydrates such as hyaluronic acid are preferable, and chitosan is more preferable in view of the supply of raw materials and cost.
Further, it is possible to introduce a group having chemically crosslinkable characteristics instead of the photoreactive group. The introduced group is preferably capable of rapid intramolecular crosslinking and easy switching thereof. Since the photoreactive group has the characteristics of easy switching, high reactivity, and few unreacted active sites remain, the photoreactive group can be suitably used. Further, chitosan, having an amino group at the second position, is also advantageous for the introduction reaction of the photoreactive group, and therefore, they are suitably used.

The composition of the present invention is characterized by containing, in addition to the above-mentioned photo-crosslinkable chitosan derivative (PRC), a saccharide and/or amino acid.
The saccharide to be used in the present invention is preferably one having relatively low-molecular weight, such as neutral monosaccharide, disaccharide or oligosaccharide including glucose, galactose, mannose and fucose. Glucose is especially favorable. When anionic saccharide is used, it may form poly-ion complex with chitosan derivative and sometimes gels without expose to light.

On the other hand, amino acid to be used in the present invention may be commonly known amino acids, such as glutamine, alkaline, serine, and the like. Although it is not limited, it is preferable to use essential amino acids (phenylalanine, leucine, valine, histidine, methionine, isoleucine, lysine, threonine, tryptophan, arginine or glycine).

The medical composition of the present invention can be prepared by resolving the photo-crosslinkable chitosan (PRC) and amino acid and/or saccharide as well as optional compositions into a solvent, preferably an aqueous medium, preferably at a neutral pH. For example, it may be prepared by adding amino acid or saccharide to a PRC solution in distilled water or phosphate buffer (PBS), or by mixing PRC solution with a cell culture medium containing amino acid and/or saccharide. The cell culture medium preferably used in the present invention is a mixed culture medium (DMEM/F12) of Dulbecco's Modified Eagle Medium (DMEM) and Ham's F12 Medium (DMDM/F12). However, even if the culture mediums for human cell line available from Research Institute for the Functional Peptides and NISSUI PHARMACEUTICAL Co., LTD or those used at the RIKEN CELL BANK are used, the desired degradation characteristic (polynudear globe infiltration characteristic) can be obtained. As for other mediums, please refer to http://func-p.co.jp/hitoseihin.htm) or http://www.brc.riken.jp/lab/cell/distribution/med_table.shtml, for example.

The content of photo-crosslinkable chitosan derivative (PRC) in the medical composition of the present invention is generally 0.01 to 100mg/ml, more preferably 1 to 50 mg/ml at least, even more preferably 5 to 30 mg/ml, and in particular 20 mg/ml, in order to secure injectability to circumference region of skin graft and support the skin graft with gel after cross-linked.
On the other hand, although the content of amino acid and/or saccharide is not limited, the desired degradation characteristic can be gained with an amino acid concentration of about 0.01 to 50 mg/ml, more preferably about 0.1 to 25 mg/ml and further preferably about 0.2 to 200 mg/ml. As for saccharide, the desired degradation characteristics can be obtained with a concentration of about 0.1 to 250 mg/ml, preferably about 1.0 to 200 mg/ml, more preferably about 1.5 to 150 mg/ml. The desired effect of addition of the amino acid and/or saccharide can be obtained by either single addition or mixed addition, as long as the PRC solution does not become insoluble before it is exposed to light.

Since the medical composition of the present invention prepared as described above includes photo-crosslinkable chitosan derivative (PRC), it forms an insoluble gel through cross-linking in a short time by irradiation with light of predetermined strength (UV or visible) for predetermined time period, so that a skin graft can be adhered.
Conditions for cross-linking by light vary according to the types and degree of substitution of the photo-reactive groups introduced into the photo-crosslinkable chitosan derivative to be used, amounts of the chitosan derivative contained in the composition and amounts of the composition to be used and the like. If predetermined accumulated light amount is attained with UV having wavelength of less than 400 nm, rapid cross-linking reaction occurs and a practical chitosan hydro-gel can be obtained.
For example, accumulated light amount of 50-300 mj/cm² can provide a good cross-linked hydro-gel formed with the subject composition, in a measurement of light amount with a 365 nm detection type illuminometer (UIT-150, USHIO INC.).
The subject composition undergoes cross-linking reaction with the UV having a wavelength of less than 400 nm from, for example, a UV-LED, excimer laser or mercury lamp, and the radiation time to obtain the required accumulated light amount can be shortened by increasing radiation intensity. It is possible to obtain the desired hydro-gel with an radiation time of 1 second or less.

The crosslinking reaction degree of the photoreactive group of the chitosan matrix is not particularly limited. In general, it is considered that there is a trend that when the crosslinking reaction degree is high, initial release of the drug is small and suitable function as a drug releasing body can be obtained. Therefore, the crosslinked chitosan matrix of the present invention has a crosslinking reaction degree of at least 30, preferably from 40 to 100%, more preferably from 50 to 100%, much more preferably from 60 to 100%, and even much more preferably from 70 to 100%. Here, "crosslinking reaction degree (or crosslinking degree)" in the present invention represents the ratio of photoreactive groups bound with other groups and the like, among the photoreactive groups existing in the photo-crosslinkable chitosan derivative.

When the medical composition of the present invention is used, it is considered that, due to the addition of glucose or amino acid to the chitosan gel layer, the distance between the chitosan molecules after the photo cross-linking reaction expands and cell infiltration becomes easier.
It is inferred that, the cell infiltration is facilitated by the addition of amino acid is due to the coating effect derived from the amino acid. That is, it can be considered that neutralization by the amino acid of the chitosan amino group that prevents infiltration of the cell, which is an acidic particle coated with sialic acid, makes the mobility of the cell easier. The addition effects which enable easier cell infiltration to the chitosan gel can be obtained even if saccharide and amino acid are used separately or used together, and the effectiveness is not reduced in both cases.

It is known that glucose and glutamic acid stimulate the proliferation of leucocyte cells. The present inventors consider that the main cause of the effects observed in the present invention is cell energy effect due to the saccharide and amino acid. Therefore, although a photo cross-linked chitosan gel is used as the base material in the following Examples, the effects of the present invention can be obtained even if the chitosan gel is replaced with another base material, such as other hydro-gel, collagen or gelatin preparation, by using it in combination with a saccharide such as glucose or an amino acid. Therefore, medical composition containing such other base materials and saccharide and/or amino acid is within the scope of the present invention.

### Examples

Detailed descriptions of the present invention will be hereinafter given by using concrete examples. However, these concrete examples do not limit the scope of the present invention.

### (Synthesis Example 1)

### Synthesis of photo-crosslinkable chitosan derivative (PRC)

PRC wherein an ultraviolet reactive group and a carbohydrate chain were introduced into a chitosan backbone structure was synthesized in accordance with the method described in WO00/27889. More specifically, azide (p-azide benzoate) and lactose (lactobionic acid) were introduced by condensation reaction into an amino group of crab-derived chitosan having 800 to 1,000 kDa of molecular weight and 85% of deacetylation degree (available from Yaizu Suisankagaku Industry Co., Ltd.). It was confirmed that the resultant was soluble in neutral pH due to introduction of lactose, and substitution degrees of p-azide benzoate and lactobionic acid were about 2.5% and 5.0%, respectively.
Further, when chitosan materials derived from crab shell and a chitosan material derived from cuttlefish cartilage were used, similar derivatives could be synthesized.

### (Synthesis Example 2)

### Synthesis of photo (visible light) cross-linkable chitosan derivatives (VL-RC)

Methyl-4-[2-(4-formylphenyl)ethenyl] pyridine methosulfonate (FPP) expressed by the following formula was synthesized in accordance with the method described in Journal of Polymer Science: Polymer Chemistry Edition, Vol. 20,1419-1432 (1982). 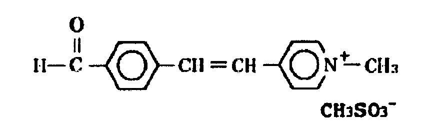 More specifically, under the condition of cooling with ice, a solution of γ-picolline (3.07 g, 33 mmol) in methanol (8.3 ml) was added to dimethyl sulfate (4.16 g, 33 mmol). After the solution was left for 1 hour at room temperatures, terephthalic aldehyde (13.4 g, 100 mmol) was added to the solution and dissolved therein by heating. Subsequently, piperidine (0.47 ml) was added, and refluxed for 5 hours. A separated substance was removed by heating filtration. A hot filtrate was mixed with a mixed solvent of ethanol (50 ml) and acetone (16.7 ml), which was left overnight at room temperature. A yellow separated substance was batched off by filtration, which was washed with ethanol and acetone, and then dried under reduced pressure to obtain FPP. Its yield was 4.81 g (46%) and its melting point was from 210 to 213°C.

### (Example 1)

Loss of full-thickness skin layers having an area of 3 cm x 3 cm was artificially created on the back of rats. The removed skin was taken as an autodermic graft specimen and 12 holes of 5mm diameter were created on the graft specimen.
The graft specimen was then placed on portions where the loss of full-thickness skin was created and results were evaluated with the following items in the cases where: (A) it was left without any actions; (B) the graft specimen was sutured; (C) a conventional medical adhesive (cyano acrylamide type, product name: Dermabond (Johnson & Johnson K.K.) was filled in the hole; and (D) the composition of the present invention was filled in the hole and then irradiated by UV (wavelength 330 nm for 15 seconds). The results are shown in Table 1.

**Table 1**

| | (A) | (B) | (C) | (D) |
|---|---|---|---|---|
| (1) Time needed for treatment (min.) | 0.8 | 10.8 | 5.5 | 7.9 |
| (2) Adhesion rate of graft specimen (%) (a) | 69.4 | 97.2 | 100.0 | 91.7 |
| (3) Granulation around graft specimen ^{(b)} | (+) | (+) | (-) | (+) |

| | | | | |
|---|---|---|---|---|
| (a) Adhesion rate: The number of graft specimen showing adhesion by macro observation at 5 days after grafting / the total number of graft specimen (N=12). (b) Granulation: (+): Formation of granulation tissue was confirmed around the graft specimen in histological observation at 7 days after grafting. (-): Formation of granulation tissue was not confirmed around the graft specimen in histological observation at 7 days after grafting. | | | | |

As Table 1 clearly indicates, adhesion rate of untreated graft specimen (A) was dramatically low, and the adhesion rate improved when the graft specimen was sutured (B), but it takes 10 minutes or more for the treatment. On the other hand, when cyano-acrylic adhesive was used (C), the treatment time and the adhesion rate are excellent, but granulation around the graft specimen was not observed. In contrast, when the composition of the present invention was used (D), treatment time, adhesion rate and granulation are excellent.

### (Example 2)

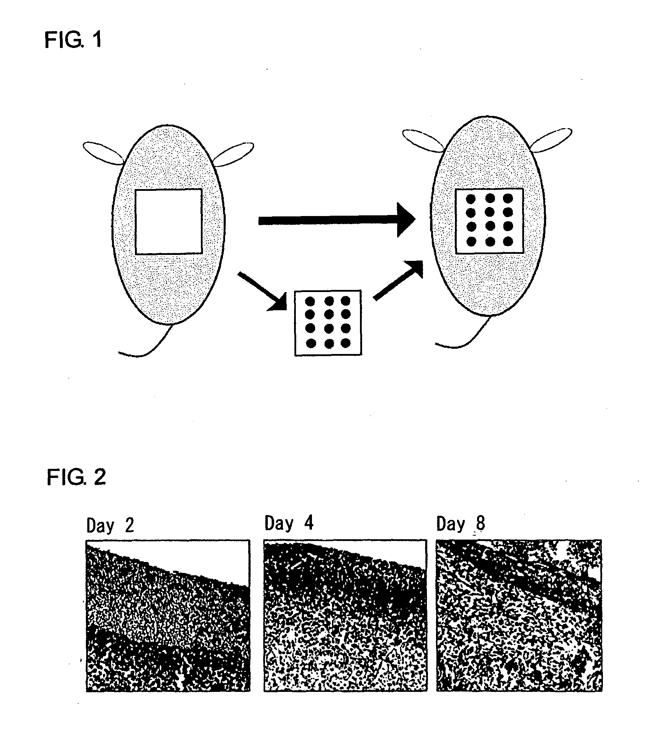

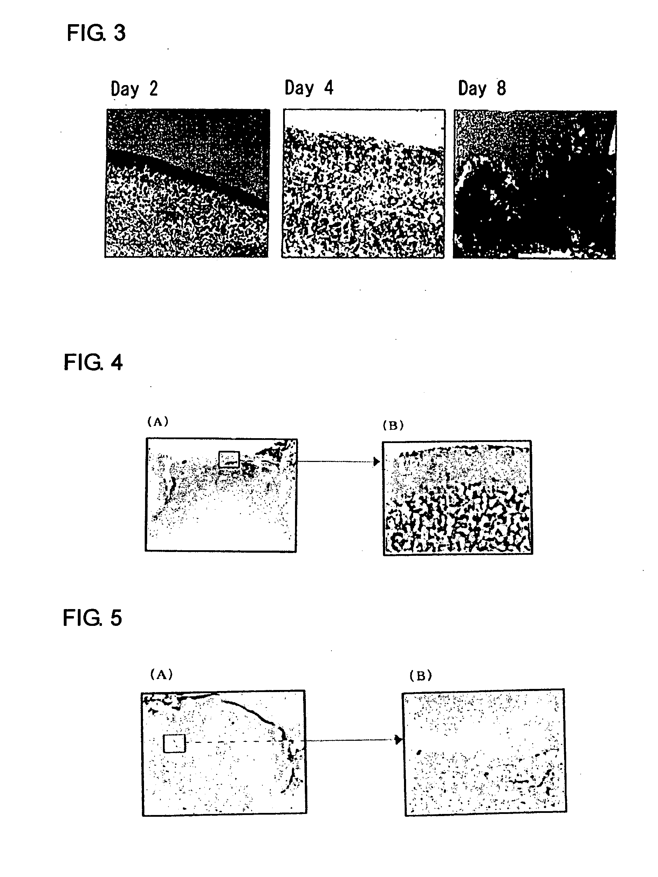

4% PRC aqueous solution (distilled water) was prepared, and mixed with the same amount of medium (DMEM/F12, Invitrogen Corporation) and the resultant was used as composition (A) according to this Example. Comparative composition (B) was prepared by mixing the same amount of PBS, instead of the above medium. The medium (DMEM/F12) used in composition (A) is a serum free tissue culture medium containing various types of amino acid, glucose and other components.
Similar to Example 1, autodermic skin graft specimen was placed on the area of loss of full-thickness skin of rats, the compositions (A) and (B) were filled thereinto and UV was irradiated. The cross-sectional tissues of the grafted portions were then examined, and the results are shown in Figures 2 and 3.

As shown in Figure 2, when the composition (A) of the present invention was used, the neutrophil were infiltrated into the chitosan layer in high density (day 2), the chitosan layer disappeared and neovascular and granulation were observed (day 4), and further, epithelialization was observed (day 8). In contrast, when composition (B), which did not contain amino acid and glucose, was used, as shown in Figure 3, no infiltration into chitosan layer was observed. Although shrinkage of the chitosan layer was seen on day 8, infiltration in tissue directly under the chitosan layer was significant (day 2) and granulation with fibroblast was active.

### (Example 3)

The same experiment as Example 2 was carried out, and the tissue containing the PRC gel layer was removed after predetermined time (4 days), and was immuno-stained with anti-VEFG antibodies. Figures 4 and 5 indicate the respective results obtained from the Example (A) using a medium containing amino acid and glucose and Comparative Example (B) using PBS.
As indicated in Figure 4, a strong stain was found inside of the PRC gel layer in Example (A) using the composition of the present invention (Figure 4(A)). Especially, fibrous layer containing many neutrophil cells existed in the upper layer of the gel, and a strong VEFG stain was observed (Figure 4(B)).
On the other hand, in Comparative Example (B), in which PRC was dissolved in PBS, decomposition of PRC gel layer was not found, and a strong stain was found at the bottom layer where the PRC gel layer contacts hypodermis (Figure 5(A)). In the interfacial region, PRC gel changed to fibrous status and a strong stain of VEGF was observed.

### (Example 4)

Composition (A), in which PRC was dissolved in PBS, Composition (B), which is obtained by adding 50 mg/ml D-glucose to Composition (A), and Composition (C), which was obtained by adding 5 mg/ml glutamine to Composition (A) were prepared. Composition (D), which is obtained by adding the same concentration of glucose and amino acid as the ones in Compositions B and C, was prepared.
Similar to Examples 2 and 3, portions where loss of full-thickness skin ware artificially created on the backs of rats, autodermic graft specimens were placed on the respective portion, and the Compositions (A), (B), (C) and (D) were applied and UV was irradiated for curing. The tissues were collected on 5 days after the treatment, and the infiltration degrees of granulocytes into chitosan gel layer were evaluated using microscopic observation. The results are shown in Table 2.

**[Table 2]**

| Infiltration degree of granulocytes into chitosan layer | |
|---|---|
| (A) | - |
| (B) | ++ |
| (C) | ++ |
| (D) | +++ |

| | |
|---|---|
| Notes: +++: Granulocytes were infiltrated into entire gel layer in high density. ++: Granulocytes were infiltrated into entire gel layer. +: Granulocytes were infiltrated only around the interfacial region between the gel layer and granular. -: Infiltration of granulocytes into the gel layer was not observed. | |

It was obvious that addition effects found in Examples 2 and 3 with the medium could be observed by adding only glucose or amino acid, separately. Furthermore, similar effective infiltration of granulocytes was observed when the amino acid added was changed to mixture of essential amino acids (Data is not shown).

### (Example 5)

Portions of loss of full-thickness skin area with 2 cm were created on the back of rats. Those portions were treated with a composition of the present invention or collagen film (TERUDERMIS (trade name); TERUMO CORPORATION), which is commercially available artificial dermis (autodermic skin grafts were not used).
On 6 days after treatment, in wound (A), which was treated with the composition of the present invention, the chitosan gel had already disappeared, granulation proceeded quickly, and epithelialization from the tissue around the wound progressed. On the other hand, in wound (B), which was treated with the collage film, epithelialization from the tissue around the wound was observed, but collagen film remained. For (A), it was not necessary to cover the chitosan gel cured by UV irradiation with a coating such as gauzes. However, for (B), it was necessary to suture the collagen film for adhering it and the wound was created after removal of the suture.

### (Example 6)

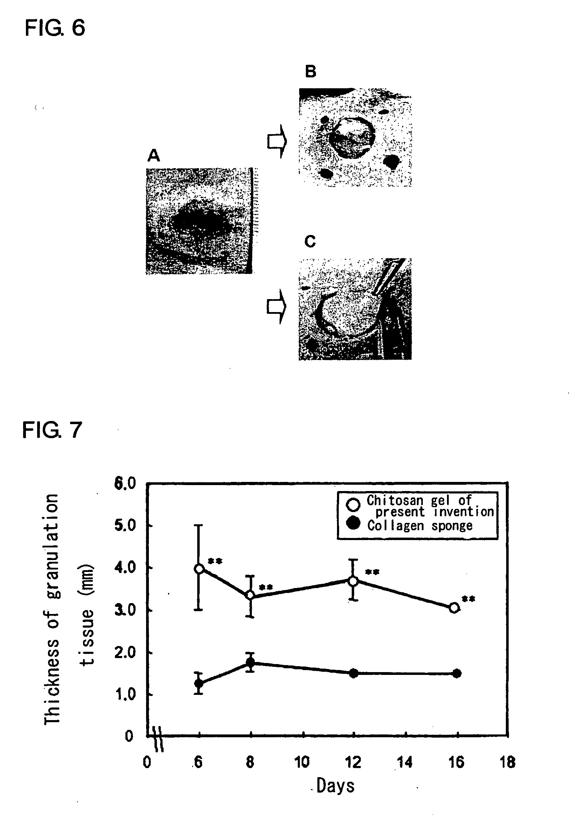

Bums of degree III were artificially created on the back of rats and necrotic tissue was removed (Figure 6A). Next, the subject areas were treated, as support materials, with the composition of the present invention (Figure 6A) or a collagen sponge (TERUDERMIS (trade name), TERUMO CORPORATION) (Figure 6C). That is, an example in which the composition of the present invention (DMEM/F12 containing PRC aqueous solution) was filled in the wound area and photo-cured, and another example in which the wound area was treated with a collage sponge were compared.

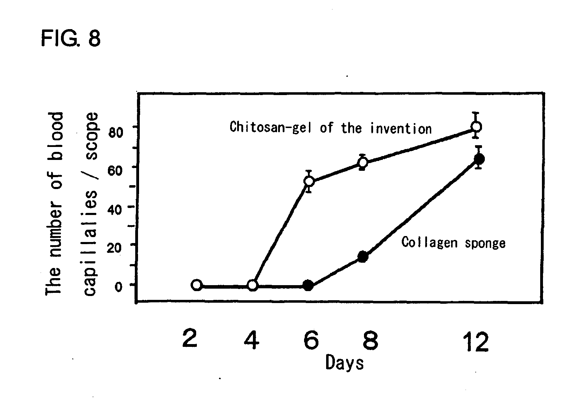

When the subject area was treated with the composition of the present invention, neovascular appearance was found after 6 days of grafting. In contrast, when the collagen sponge was used, the blood flow low on day 6, and the neovascular appearance reached its peak after the 12th day after grafting. Figures 7 and 8 show the granulation tissue thickness on the wound area and the change of the number of blood capillaries observed using microscopy. It was found that the composition of the present invention highly facilitated granulation and angiogenesis.

The epithelium thickness on 32 days after grafting reached to 67.1 µm in average in the example treated with the composition of the present invention, but was 55.8 µm in average in the example treated with collagen sponge.
Accordingly, from the results of these examples, by using the medical composition of this invention, surprising results were obtained, those are granulocyte infiltration into the chitosan gel layer, followed by an increase of angiogenesis and moreover induction of epithelialization, without using autodermic cells.

## Claims

1. Medical composition comprising a base material and an amino acid and/or a saccharide.

2. Composition according to claim 1, wherein the base material is selected from hydro-gel, hydrocolloid, collagen, and gelatin preparations.

3. Composition according to claim 1, wherein the base material is composed of photo-crosslinkable chitosan derivative.

4. Composition according to claim 1, wherein the saccharide is a neutral saccharide selected from glucose, galactose, mannose and fucose

5. Composition according to claim 1, wherein the amino acid is essential amino acid.

6. Composition according to Claim 3, wherein the photo-crosslinkable chitosan derivative is a polymer obtainable by incorporating a carbohydrate chain containing a reducing terminal to at least one part of amino groups of the glucosamine units (1) and incorporating a photoreactive group to at least another part of amino groups of the glucosamine units of a chitin/chitosan having the constituent units expressed by the following formulas (1) and (2).

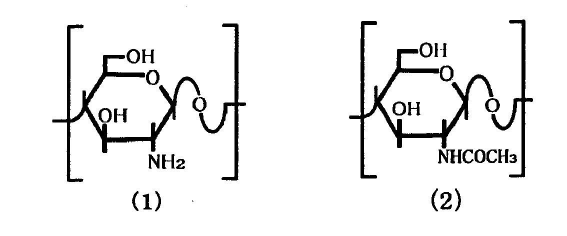

7. Composition according to claim 3, wherein it contains at least 1 mg/ml of the photo-crosslinkable chitosan derivative.

8. Composition according to any one of claims 1 to 7, wherein it further comprising a wound healing promoter.
